# EUROPEAN PATENT APPLICATION

(11) **EP 4 725 424 A1**
(43) Date of publication of application: **15.04.2026**
(21) Application number: 24205632.3
(22) Date of filing: 09.10.2024
(51) Int. Cl.: A61B 10/00

(54) **INTRA-VAGINAL MEASURING DEVICE**

(71) Applicant: Abele, Hermann, 97261 Güntersleben (DE)
(72) Inventor: Abele, Hermann, 97261 Güntersleben (DE)
(74) Representative: Flach Bauer & Partner Patentanwälte mbB

(57) **Abstract**

The present invention relates to an intra-vaginal measuring device 100. The measuring device 100 is substantially ring shaped and configured to be placed around a mammal's cervix. The measuring device 100 includes a sensor unit and an active magnetic communication unit 120. The sensor unit 110 is configured to measure at least one physiological parameter being indicative for the fertility status of the mamma. The active magnetic communication unit 120 is configured to transmit data 20 being based on the at least one measured physiological parameter from inside the vagina to a receiving base station 200 outside the mammal's body.

## Description

### Field of the invention

The present invention relates to an intra-vaginal measuring device, to an intra-vaginal measuring intra-vaginal measuring system including said device and to a method for determining a fertility status of a mammal using said intra-vaginal measuring device, particularly for contraception or natural family planning.

### Background

Contraceptive methods and devices have evolved significantly over time, offering individuals and couples a wide array of choices for controlling reproductive outcomes. Contraception devices are designed to prevent pregnancy through various mechanisms, primarily by inhibiting fertilization or implantation.

Mechanical contraception devices, commonly referred to as barrier devices, physically block sperm from reaching the egg. The most prominent examples include condoms and diaphragms. Those barrier devices can be combined with e.g. spermicides, to inactivate sperms being present in the vagina. Further, intrauterine devices (IUDs) are known. Those can be copper-based or hormonal.

Medical contraception involves the use of hormonal or biochemical agents to prevent pregnancy. The most common forms include oral contraceptive pills, hormonal implants, contraceptive injections, patches, and vaginal rings. Oral contraceptives, widely known as "the pill," contain synthetic hormones (either a combination of estrogen and progestin or progestin alone) that prevent ovulation, thicken cervical mucus, and thin the uterine lining, thus inhibiting fertilization and implantation.

Further, the advent of digital technology has led to the development of digital contraception, often referred to as fertility tracking or fertility awareness-based methods (FABMs). These devices leverage modern sensors and algorithms to monitor physiological indicators such as basal body temperature, hormone levels, and menstrual cycle data, predicting a woman's fertile window and suggesting when to abstain from intercourse or use alternative contraception methods to avoid pregnancy. Examples of these devices include smartphone apps, wearable devices, and digital thermometers.

One advantage of digital contraception devices is their non-invasive nature, making them a natural choice for those seeking to avoid hormonal or mechanical methods. These devices also provide valuable insight into reproductive health, enabling women to gain a deeper understanding of their cycles. Moreover, they are entirely reversible and do not interfere with natural body processes.

However, present digital contraception devices are not without limitations. Their effectiveness relies heavily on user diligence and accuracy in tracking physiological data. The failure rate of fertility awareness-based methods is higher than that of other methods, especially when users do not strictly follow the guidance provided by the device. Additionally, the accuracy of predictions can be affected by irregular menstrual cycles or external factors such as stress, illness, sports, or medication, which may alter temperature or hormone levels.

Thus, known digital contraception devices are recommended to be accompanied by manual cervix mucus evaluation. This is not only time consuming but also inconvenient, as the cervix mucus is typically judged haptically.

Further, in known digital contraception devices, physiological data - which is very sensitive data - is oftentimes transferred to a smart phone or other mobile device, using communication paths, such as Bluetooth or WiFi.

Those communication paths have a high transmission range, making them prone to eavesdropping, particularly in case security vulnerabilities become known in the communication path. Thus, regular updates may become necessary.

Further, those communication paths use frequencies in the GHz range. These GHz-frequencies tend to be blocked by tissue. Thus, digital contraception devices that are operated inside the body, e.g. intra vaginal, need to be removed for data transmission (i.e. at least once a day), making them inconvenient to use. Further, the required removal and reinsertion requires the user to be compliant. If not, the effectiveness and reliability may be significantly reduced.

### Summary of the invention

In view of the above, the object of the present invention is to provide a device, system and method for contraception or natural family planning that overcome the aforementioned drawbacks at least partially. Particularly, a more reliable and safe communicating intra-vaginal measuring device shall be provided.

The object is achieved by an intra-vaginal measuring device according to claim 1 and by a base station, a system and a method according to the subordinate claims. Further aspects of the invention are given in the dependent claims as well as in the following description.

In particular, the object is achieved by an intra-vaginal measuring device. The intra-vaginal measuring device is substantially ring shaped and configured to be placed around a mammal's cervix. The intra-vaginal measuring device may be designed for humans or for veterinary use.

The measuring device is not limited to contraception, but may include determination of the fertile days in order to organize reproduction naturally or through artificial insemination.

The ring shape may be a closed ring shape, therefore encircling the cervix entirely, or the ring shape may be an open ring shape, such as a C-shape. It has shown that the wearing position around the cervix is convenient and does not affect daily live. Thus, the intra-vaginal measuring device can be worn for multiple days, without the need to be removed. In an aspect, the intra-vaginal measuring device can stay in place for at least one menstrual cycle. The human menstrual cycle is about 23 to 35 days (average 28 days). Thus, the intra-vaginal measuring device may be configured to stay in place for a period of about 23 to 40 days, or of about 25 to 35 days, or of about 28 to 30 days (at least in case of a human).

In a particular aspect, the intra-vaginal measuring device may be classified as a "class IIa"-medical device according to Regulation (EU) 2017/745, i.e. a device that generally is to be installed within the body for between 60 minutes and 30 days.

After usage, the intra-vaginal measuring device may be disposed (single-use device), or may be prepared for reuse. The preparation for reuse, may include charging and cleaning the intra-vaginal measuring device.

The outer material of the intra-vaginal measuring device may be a biocompatible, flexible material including, but not limited to silicone elastomers, thermoplastic elastomers, Polyethylene Vinyl Acetate (PEVA), polyurethane, and/or the like.

The outer material encapsulates further units of the intra-vaginal measuring device at least partially. Those further units include at least a sensor unit and an active magnetic communication unit. Those units may be spread around the ring shape and/or may be provided in a thickened portion of the ring. **The** sensor unit of the intra-vaginal measuring device may include electrodes, particularly gold plated electrodes, which may be in direct contact with the inter-vaginal environment.

The sensor unit is configured to measure at least one physiological parameter being indicative for the fertility status of the mammal. In a particular aspect, the sensor unit is configured to measure multiple (at least two) physiological parameters being indicative for the fertility status.

The intra-vaginal measurement is advantageous, as it is not dependent on user diligence and accuracy. Rather, the measurement can be performed automated once the intra-vaginal measuring device is set in place. In particular, the measurement can be done at defined points in time and/or defined time intervals. For example, measurements can be done every second, every minute, every ten minutes, every hour and/or at any desired time interval. The measurement data can be processed, stored and/or transmitted.

Further, intra-vaginal measurements are less prone to environmental influences, such as outside temperature. Thus, measurement accuracy is increased compared to fertility tracking using wearables, such as smart watches, or mobile device, such as smart phones.

The measured data, being processed or raw data, can be transmitted via the active magnetic communication unit to a receiving base station outside the mammal's body. The transmission may be done at defined points in time (e.g. at 4 am) and/or defined time intervals (e.g. every hour). Further, the transmission may be done upon request, e.g. initiated by a transmission request transmitted by the base station.

Particularly, the active magnetic communication unit is configured to transmit data being based on the at least one measured physiological parameter from inside the vagina to the receiving base station. The base station may be in active magnetic communication with one intra-vaginal measuring device (e.g. in human use) or with multiple intra-vaginal measuring devices (e.g. in a stable, in veterinary use). Thus, fertility of one or more mammals can be monitored. In case there is active magnetic communication with multiple intra-vaginal measuring devices, respective magnetic inductive antennas can be placed in close proximity to the mammals to be monitored (e.g. at least one antenna per box in a stable).

Active magnetic communication is a type of wireless communication technology that leverages magnetic fields for data transmission over short distances, typically within a meter or sub-meter range. In an aspect of the present invention, the active magnetic communication unit may be designed so as to have a maximum transmission range being in a range from 50 cm to 120 cm, or in a range from 70 cm to 110 cm, or in a range from 80 cm to 100 cm. This makes the wireless communication less prone to eavesdropping. The transmission range is inter alia dependent on the transmission power and the antenna design.

Unlike far-field communication methods (such as Wi-Fi or Bluetooth), which rely on electromagnetic waves, magnetic communication operates by inducing a magnetic field in one device (i.e. the intra-vaginal measuring device) and coupling it with another device (i.e. the base station) within close proximity.

Active magnetic communication relies on actively generated magnetic fields (rather than passive ones like in RFID) to establish a communication link. There are no outgoing electrical fields (and accordingly no electromagnetic waves) used for communication.

The intra-vaginal measuring devices, respectively the active magnetic communication unit thereof generates a magnetic field using a magnetic inductive antenna (e.g. a coil) by passing an electric current through it. This magnetic field carries encoded information, i.e. the data to be transmitted.

The oscillating magnetic field propagates, though unlike radio frequency electromagnetic waves, its range is usually shorter and less affected by environmental factors like tissue.

A receiving antenna (e.g. a coil) that is connected to the base station converts the oscillating magnetic field, respectively received magnetic fluctuations back into electrical signals. The received signal is typically actively amplified prior to further processing.

Hence, the active magnetic communication link typically requires active magnetic inductive antennas on the transmitting and the receiving side.

The active magnetic communication link may have very little stray field losses, which may be <1% of the transmission power, or even < 0,5% of the transmission power. In an exemplary embodiment, the transmission power of the active magnetic communication unit may be in a range from 0.5 to 5 mW, or in a range from 0.8 to 3.2 mW.

This active magnetic communication method is advantageous, as unlike radio frequency signals in the GHz range, it is not blocked by body tissue.

In an aspect, the active magnetic communication unit operates at frequencies in the range of 9 MHz to 14 MHz, or in a range of about 13 MHz. At these frequencies, higher data rates can be achieved. For example, the data rate may be at least 300 kbps, or at least 500 kbps, or at least 700 kbps.

In another aspect, the active magnetic communication unit may operate at frequencies in the range of tens to hundreds of kilohertz.

In an aspect, the active magnetic communication unit may be operated in a magnetic resonant coupling mode. Magnetic resonant coupling enhances the efficiency and range of the active magnetic communication by using resonance between the transmitting and receiving antennas (e.g. coils). This method allows for more efficient data communication over slightly longer distances compared to traditional magnetic inductive communication.

The active magnetic communication unit offers a robust, secure and short range communication link. Further, the active magnetic communication unit has a low power consumption. Thus, the intra-vaginal measuring device can remain in operation for a long period of time (e.g. about30 days).

Even further, as the magnetic field is used for communication, SAR-values are very low, wherein the SAR-value denotes the specific absorption rate (SAR), which is a measure of the rate at which energy is absorbed per unit mass by a human body when exposed to a radio frequency (RF) electromagnetic field.

In an aspect of the invention, the sensor unit may be configured to measure intra-vaginally at least one of the following physiological parameters:
- a body temperature
- a pH-value of the vaginal environment
- a dielectric constant of the cervix mucus
- a frequency dependent impedance of the cervix mucus
- a polarity of the cervix mucus
- a magnetic permeability of the cervix mucus
- at least one optical property of the cervix mucus, such as transmittance, reflectance, absorption, color and/or refraction
- a viscosity of the cervix mucus
- a heat conductivity of the cervix mucus
- a heat capacity of the cervix mucus, and/or
- an oxygen content of the mucous membrane.

It has been shown that these parameters naturally fluctuate during the menstrual cycle, depending on the phase of the menstrual cycle. Thus, through continuous measurement and monitoring, the menstrual cycle can be tracked, and a fertility status can be determined. The more parameters that are incorporated (sensor fusion), the more accurate the determination of the fertility status is, as interfering influences and/or erroneous measurements can be detected and taken into account when assessing the fertility status.

The intra-vaginal body temperature may be measured, using a resistance temperature detector, a semiconductor temperature sensor, and/or the like.

The pH-value of the vaginal environment may be measured, using a pH electrode and a reference electrode. Further, an ion-sensitive field-effect transistor, an antimony electrode, a polymer-based pH sensor and/or the like may be used.

The dielectric constant of the cervix mucus can e.g. be determined capacitively, e.g. using an LCR-sensor, by utilizing a resonant cavity or via time-domain reflectometry, TDR.

The impedance of the cervix mucus can be measured (frequency dependent), using an EIS (electrochemical impedance spectroscope sensor, or a bio impedance sensor. A LCR meter, a capacitive impedance sensor and/or the like can also be used. Further, a magnetic inductive sensor may be used. This type of sensor may utilize a coil that is also used for active magnetic communication. In a particular aspect, the impedance of the cervix mucus is determined by a four-point measurement.

Measuring a polarity of the cervix mucus involves determining the distribution of electrical charge or dipole moments within the cervix mucus. The dipole moment is a direct indicator of the polarity of the cervix mucus. It can be determined via the dielectric constant. Alternatively, an IR-sensor and/or the like can be used.

The magnetic permeability of the cervix mucus may be determined by an inductance measurement, e.g. utilizing an LCR meter and a coil, such as the coil being used for active magnetic communication.

For measuring optical properties of the cervix mucus, such as transmittance, reflectance, absorption, color and/or refraction, an optical sensor and optionally a light source may be provided. The optical sensor may be configured to measure the optical properties of the cervix mucus wavelength dependent. For example, the light source may be configured to emit light of a specific wave length during the measurement performed by the optical sensor.

The viscosity of the cervix mucus can e.g. be measured, based on a dampening of an ultrasound signal and/or rheology.

The heat conductivity and /or heat capacity of the cervix mucus could e.g. be measured using a heat flow meter.

Further, the intra-vaginal measuring device may include an oxygen sensor, particularly an optical oxygen sensor for measuring the oxygen content of the mucous membrane.

In a further aspect, the sensor unit is configured to measure additional physiological or fitness parameters, including e.g. a heart rate, a blood oxygen saturation (SpO₂), a motional activity, a heart rate variability (which is indicative for a stress level), a respiratory rate, and/or the blood pressure.

Those additional parameters can be considered, when judging the physiological parameter being indicative for the fertility status of the mammal. This is advantageous, as inter-vaginal body temperature increases not only due to an ovulation, but also due to motional activity, stress, and/or the like. If e.g. a motional activity or stress is detected, the motional activity / stress can be quantified and taken into consideration, when judging the physiological parameter. Here, again, the more different measurement parameters are taken into account, the more accurate the prediction of the fertility status becomes.

The intra-vaginal measuring device may include a microcontroller unit, MCU. The MCU may be configured to judge the measured physiological parameters being indicative for the fertility status of the mammal to predict a fertility status. Hence, the measured parameters may be processed and judged by the intra-vaginal measuring device. Thus, the amount of data that has to be transmitted via the active magnetic communication unit can be reduced. Further, the MCU may take into account additionally measured physiological or fitness parameters to adjust the fertility status prediction.

It is to be understood, that the transmitted data may include the measured data. In this case, the judging and/or fertility status prediction may be performed at the base station, which may have higher processing power than the intra-vaginal measuring device. In a further aspect, the transmitted data may be forwarded by the base station to a mobile device or an external computing device, wherein the judging and/or fertility status prediction may be performed on said mobile device and/or external computing device.

The intra-vaginal measuring device may include a data storage unit, wherein the data storage unit may be configured to store measured data and/or a predicted fertility status. As the data storage unit stores measured data and/or a predicted fertility status, there is no need to carry the base station along. Instead, the base station can be left at home, e.g. in a bedroom. In case the intra-vaginal measuring device is in vicinity to the base station stored data can be transmitted. This can be done e.g. upon a request issued by the base station, and/or at a predetermined time and/or at predetermined intervals.

For powering the intra-vaginal measuring device, the intra-vaginal measuring device may include a power storage unit, such as a battery. Said power storage unit may be rechargeable. In an aspect, the intra-vaginal measuring device may include an inductive charging unit for wirelessly recharging the power storage unit. Particularly, a magnetic inductive antenna (e.g. a coil) used for active magnetic communication may be used for inductively charging the intra-vaginal measuring device. In another aspect, a separate coil may be provided for wirelessly recharging the power storage unit.

Further, the intra-vaginal measuring device may be a single-use device, which is deposed after usage. In an alternative aspect, the intra-vaginal measuring device may be a multiuse device, which can be re-inserted into the vagina after e.g. the menstruation to monitor a further menstrual cycle.

In an aspect of the invention, a measuring rate of the sensor unit is increased with advancing first menstrual cycle half and/or the measuring rate is reduced after having detected ovulation. This allows to save energy, while still determining the fertile days with high accuracy.

Further, the intra-vaginal measuring device may include a magnetic inductive antenna. In an aspect, the magnetic inductive antenna may be arranged circumferentially within the ring shape of the intra-vaginal measuring device. This allows to provide relatively large coils, facilitating the magnetic inductive coupling with the base station.

In another aspect, the magnetic inductive antenna may not be arranged circumferentially, but e.g. in the thickened portion of the ring. For example, the magnetic inductive antenna may be a coil having a diameter in the range from 1 mm to 60 mm. Further, the coil may include a ferrite element.

The magnetic inductive antenna, which is used for data transmission via the active magnetic communication link may also be used for wirelessly recharging the power storage unit of the intra-vaginal measuring device.

The object is further achieved by a base station for determining a fertility status. The base station includes an active magnetic communication unit, which is configured to receive data transmitted from the active magnetic communication unit of the intra-vaginal measuring device. The active magnetic communication unit may be configured for bi-directional communication, i.e. for receiving and transmitting data.

Further, the base station includes a microcontroller unit, wherein the microcontroller unit is configured to judge physiological parameters being received with the received data to predict a fertility status. The microcontroller unit may optionally take into account additionally measured physiological or fitness parameters and/or environmental data gathered from a mobile device and/or a wearable to adjust the fertility status prediction.

Further, the base station may be configured to display measured parameters and/or a fertility status. Alternatively, or additionally, the base station may be configured to forward the received data and/or a fertility status to at least one mobile device, a wearable and/or to a cloud based storage as will be explained in greater detail below. The communication link between the base station and a mobile device, a wearable and/or a cloud based storage may include WiFi, Bluetooth and/or the like. Particularly, the communication between the base station and a mobile device and/or the communication between the base station and a wearable and/or the communication between the base station and a cloud based storage may be bidirectional.

In a further aspect, the base station may include an inductive charging unit for wirelessly recharging the power storage unit of at least one intra-vaginal measuring device.

The object is further achieved by an intra-vaginal measuring system, which system includes at least one intra-vaginal measuring device as described above and at least one base station. In an aspect, the base station is wearable, e.g. on a bracelet.

The base station includes an active magnetic communication unit, which is configured to receive data transmitted from the active magnetic communication unit of the intra-vaginal measuring device. In an aspect, the active magnetic communication may be bi-directional. Using the base station allows for a reliable and secure communication with the intra-vaginally placed measuring device.

In an aspect, the base station includes a microcontroller unit. The microcontroller unit of the base station may be configured to judge physiological parameters being received with the received data and/or to predict a fertility status. Further, the microcontroller unit may take into account additionally measured physiological or fitness parameters to adjust the fertility status prediction. Those additional physiological or fitness parameters may be measured by the intra-vaginal measuring device. Alternatively, or additionally, the microcontroller unit may take into account physiological parameters, fitness parameters, and/or environmental data gathered from a mobile device and/or a wearable to adjust the fertility status prediction.

In particular, the base station may be suited to communicate with a mobile device and/or a wearable using RF-based communication techniques, such as Bluetooth, Wi-Fi, and/or the like.

In an additional aspect, the base station may be configured to display measured parameters and/or a fertility status. In the simplest case, an indicator such as an LED is used to show whether the mammal is currently fertile or not.

Likewise, displays and graphical user interfaces can be used for displaying. For example, the progress of the fertility status and/or individual parameters may be displayed. This allows the user to better understand her own menstrual cycle.

Alternatively, or additionally, the base station may be configured to forward the received data and/or a fertility status to at least one mobile device, to at least one wearable and/or to a cloud based storage. The mobile device or a wearable may be used to display the results.

In case the results are forwarded to more than one mobile device, wearable and/or to a cloud based storage couples can jointly monitor the fertility for equal rights to family planning. Further, physicians or veterinarians can be involved in the monitoring, for example, to schedule an artificial insemination or hormone therapy.

In an aspect, the base station may include an inductive charging unit for wirelessly recharging the power storage unit of at least one intra-vaginal measuring device. Thus, multi-use intra-vaginal measuring devices can be easily recharged.

The object is further achieved by a method for determining a fertility status of a mammal. This method utilizes an intra-vaginal measuring device as described above. The method includes the following:
- determining, using the sensor unit of intra-vaginal measuring device that the intra-vaginal measuring device is intra-vaginally inserted. This can e.g. be determined by measuring a temperature.
- measuring, using the sensor unit at least one physiological parameter being indicative for the fertility status of the mammal and optionally measuring additionally at least one physiological or fitness parameter. In an example, temperature, pH-value and conductivity of the cervix mucus is measured periodically (e.g. every 10 minutes).
- transmitting, using the active magnetic communication unit data being based on the at least one measured physiological parameter from inside the vagina to a receiving base station outside the mammal's body. The data may be raw data, i.e. the measured parameters, or may be processed. In case the data is processed, it may contain a present fertility status.

The method may further comprise at least one of the following:
- judging, by the intra-vaginal measuring device, the measured physiological parameters being indicative for the fertility status of the mammal to predict the fertility status, and optionally taking into account additionally measured physiological or fitness parameters to adjust the fertility status prediction,
- judging, by the base station physiological parameters being received with the received data to predict a fertility status, and optionally taking into account additionally measured physiological or fitness parameters and/or environmental data gathered from a mobile device and/or a wearable to adjust the fertility status prediction, and/or
- displaying measured parameters and/or a fertility status via the base station, at least one mobile device and/or a wearable.

### Brief description of the figures

Further features and advantages will be apparent from the following description of exemplary embodiments, which are not to be understood as limiting, as well as the accompanying drawings, to which reference is made. In the drawings:
- Fig. 1: schematically illustrates the female menstrual cycle;
- Fig. 2: shows schematically an intra-vaginal measuring device;
- Fig. 3: shows schematically a measuring intra-vaginal measuring system, and
- Fig. 4: shows schematically a measuring contraception method.

### Detailed description of the figures

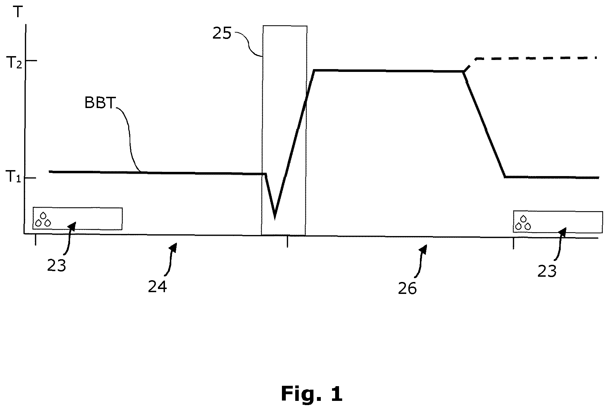

Fig. 1 schematically illustrates the female menstrual cycle. Particularly a temperature graph of the basal body temperature is given. However, other physiological parameters are subject to natural variations during the cycle.

Detailed knowledge of these physiological parameters can be used to determine the fertility status and in particular the fertile days. Depending on family planning, sexual intercourse can be avoided during these fertile days or contraceptives such as condoms can be used. It is also possible to consciously use the fertile days for family planning, either naturally or through artificial insemination.

As it is generally known, the female menstrual cycle consists of four distinct phases: the menstrual phase, follicular phase, ovulation, and luteal phase. These phases are regulated by hormonal changes that affect various physiological parameters (such as bio-physical and bio-chemical markers), including but not limited to basal body temperature (BBT), cervical mucus, and its parameters, such as optical appearance, pH, and conductivity.

The menstrual phase 23 (about days 1-5, in humans) begins with the shedding of the uterine lining due to a drop in progesterone and estrogen levels. During menstruation, the basal body temperature BBT is typically at its lowest point T₁. Further, during menstruation the vaginal environment is acidic, with a pH of around 3.8 to 5.0. The cervix produces little to no mucus, and any present is thick and opaque. The optical clarity of mucus is low. The electrical conductivity is low, because of its dense consistency.

Menstruation is followed by the follicular phase 24 (about until day 13, in humans). During this phase estrogen levels begin to rise as follicles in the ovaries mature. The basal body temperature BBT remains relatively low and steady throughout the follicular phase.

As estrogen increases, cervical mucus production rises, becoming more fluid and transparent. The optical clarity improves, and the mucus becomes "egg-white" in consistency as ovulation approaches.

Further, during the follicular phase the pH of cervical mucus becomes more alkaline, rising to around 6.5-7.5 to create a sperm-friendly environment. Conductivity also increases, reflecting the increased water content and higher electrolyte levels, which correlate with fertility.

The follicular phase 24 is followed by ovulation 25 (about day 14, in humans). A surge in luteinizing hormone (LH) triggers ovulation, where a mature egg is released. Just before ovulation, a minor temperature drop can be observed. Immediately following ovulation, BBT increases by about 0.3-0.5°C due to rising progesterone levels.

At ovulation, cervical mucus is at its peak volume and is highly transparent, resembling raw egg whites. Its optical clarity is maximal due to its high water content, allowing for better sperm motility. The mucus during ovulation remains alkaline or gets even more alkaline. The pH is about 7.0-8.0 to support sperm survival. Conductivity peaks during ovulation, reflecting the increased ion concentration and fluidity of the mucus.

Subsequently, the luteal phase 26 (about days 15-28, in humans) begins. The luteal phase 26 is dominated by progesterone, preparing the uterine lining for possible implantation. In the luteal phase 26 basal body temperature BBT stays elevated due to progesterone's thermogenic effects. If pregnancy does not occur, BBT will drop just before menstruation 23 begins (solid line). If there is a pregnancy, BBT remains at high levels (dotted line).

After ovulation, cervical mucus decreases in quantity and becomes thick, sticky, and less transparent. Its optical properties become cloudier as water content decreases. The pH becomes more acidic again (around 4.0-6.0) due to decreased estrogen, creating a less favorable environment for sperm. Conductivity also decreases, reflecting the thicker consistency and reduced ion concentration in the mucus.

Summarizing, throughout the menstrual cycle, basal body temperature, cervical mucus consistency, optical clarity, pH, and conductivity (inter alia) fluctuate in response to hormonal changes. BBT remains low during the follicular phase, rises post-ovulation, and decreases before menstruation. Cervical mucus evolves from thick and opaque to clear and slippery at ovulation, with pH and conductivity peaking to enhance fertility. These physiological changes can be used in fertility tracking and reproductive health monitoring.

In order to make reliable predictions about (in)fertility, these parameters must be measured and recorded accurately. In addition, interfering factors should be identified and taken into account when evaluating the data to avoid drawing false conclusions that could lead to an unwanted pregnancy, for example.

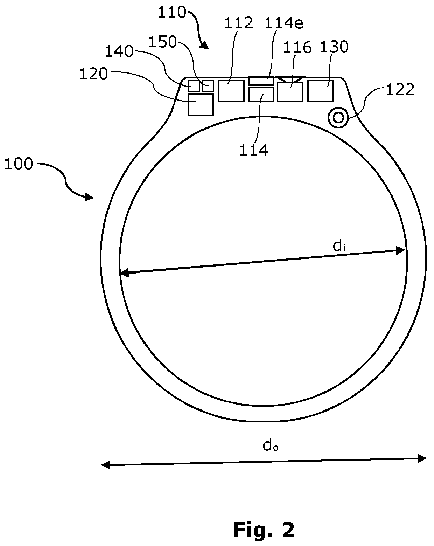

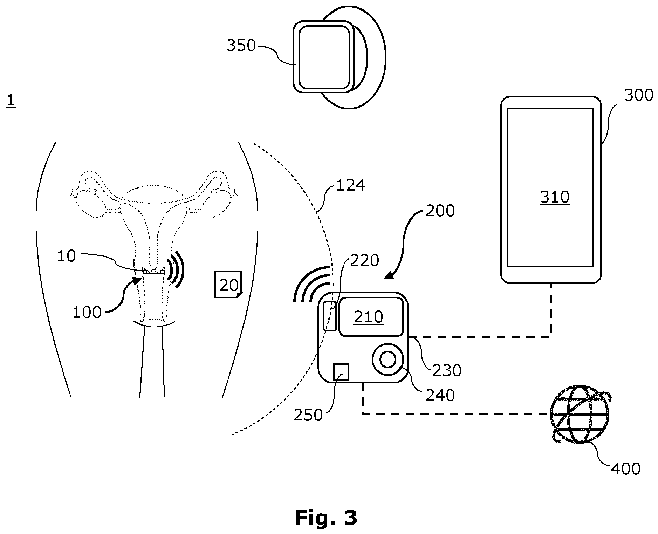

An accurate (in)fertility prediction can be achieved with an intra-vaginal measuring device, as shown in Fig. 2 and a respective system as shown in Fig. 3.

Fig. 2 shows schematically an intra-vaginal measuring device 100. The measuring device 100 is substantially ring shaped and configured to be placed around a mammal's cervix 10, as shown in Fig. 3. The ring shape has an inner diameter dᵢ and an outer diameter dₒ. The outer diameter dₒ may be in a range from 45 mm to 60 mm, or in a range from 50 mm to 56 mm. The inner diameter dᵢ may be in a range from 37 mm to 52 mm, or in a range from 42 mm to 48 mm. Here, the units of the intra-vaginal measuring device are provided in a lateral thickening of the ring shape.

The measuring device 100 includes a sensor unit 110 having in the embodiment shown multiple sensors. Here, the sensor unit 110 may include a temperature sensor 112 for measuring the intra-vaginal temperature, a cervix mucus-sensor 114, such as a pH sensor and/or a sensor for measuring a conductivity. This sensor may include an electrode 114e, which is arranged so as to come into contact with cervix mucus. Further, an optical sensor 116 is optionally provided. This optical sensor 116 may be used for measuring at least one optical property of the cervix mucus, such as transmittance, reflectance, absorption, color and/or refraction. Further, the optical sensor 116 may e.g. be suited for measuring an oxygen content of the mucous membrane.

For powering the intra-vaginal measuring device 100 a power storage unit 130, such as a (rechargeable) battery is provided. For transmitting data to a base station, the intra-vaginal measuring device 100 includes an active magnetic communication unit 120. The active magnetic communication unit may operate at a frequency of about 13 MHz and may be coupled to a magnetic inductive antenna 122, such as a coil. The coil may be arranged in the thickening (as shown) or may be arranged circumferentially within the ring shape of the intra-vaginal measuring device 100.

Further, the intra-vaginal measuring device 100 may include a data storage unit 140. The data storage unit 140 being configured to store measured data and/or a predicted fertility status.

Still further, intra-vaginal measuring device 100 includes a microcontroller unit, MCU, 150. The MCU 150 is configured to judge the measured physiological parameters being indicative for the fertility status of the mammal to predict a fertility status. Additionally, the MCU 150 may take into account additionally measured physiological or fitness parameters to adjust the fertility status prediction.

Fig. 3 shows schematically a measuring intra-vaginal measuring system 1. The system includes an intra-vaginal measuring device 100, which is already inserted into a mammal's vagina and placed around the cervix 10.

The system 1 includes a base station 200, which is in active magnetic communication with the intra-vaginal measuring device 100, allowing data 20 to be transmitted from the intra-vaginally placed measuring device 100 to the base station 200, which is located outside the mammal's body. As shown, the active magnetic communication is limited in range. The maximal transmission range 124 is about 100 cm. The active magnetic communication unit 220 of the base station and the active magnetic communication unit 120 of the intra-vaginal measuring device 100 are designed accordingly.

The base station 200 further includes a display 210 for displaying measured parameters and/or a fertility status. Still further, the base station 200 includes a microcontroller unit MCU 250. The MCU 250 is configured to judge physiological parameters being received with the received data 20 to predict a fertility status.

Further, the MCU 250 may take into account additionally measured physiological or fitness parameters to adjust the fertility status prediction. Those additional physiological or fitness parameters may be measured by the intra-vaginal measuring device 100. Alternatively, or additionally, the MCU 250 may take into account physiological parameters, fitness parameters, and/or environmental data gathered from a mobile device 300 and/or a wearable 350 to adjust the fertility status prediction.

For communicating with the mobile device 300, such as a smart phone or tablet, or a wearable 350 the base station 200 includes a wireless communication interface, for example Bluetooth (e.g. BLE) or Wi-Fi. This communication interface 230 may be used to forward the received data and/or a fertility status to at least one mobile device 300, to a wearable 350 and/or to a cloud based storage 400. The mobile device 300 or a wearable 350 may be used to display the results (e.g. a fertility status). Accordingly, the mobile device 300 and/or the wearable may have a display 310.

In an additional aspect, the base station 200 may include an inductive charging unit 240, allowing to recharge the measuring device 100, after being removed from the vagina.

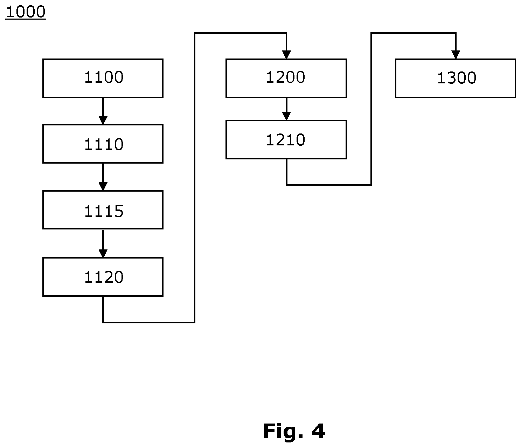

Fig. 4 gives a schematic flow diagram of a method 1000 for determining a fertility status of a mammal. This method 1000 utilizes an intra-vaginal measuring device 100 as shown in Fig. 2. The method includes
- determining 1100, using the sensor unit 110 of intra-vaginal measuring device 100, that the intra-vaginal measuring device 100 is intra-vaginally inserted. This can e.g. be determined by measuring a temperature.
- measuring 1110, using the sensor unit 110 at least one physiological parameter being indicative for the fertility status of the mammal and optionally measuring additionally at least one physiological or fitness parameter. In an example, temperature, pH-value and conductivity of the cervix mucus is measured periodically (e.g. every 10 minutes) via the sensors 112, 114.
- Transmitting 1120 and receiving 1200, using the active magnetic communication units 120, 220 data 20 being based on the at least one measured physiological parameter from inside the vagina to a receiving base station 200 outside the mammal's body. The data 20 may be raw data, i.e. the measured parameters, or may be processed. In case the data 20 is processed, it may contain a present fertility status.

The method 1000 may further comprise at least one of the following:
- judging 1115, by the intra-vaginal measuring device 100, particularly by the MCU 150, the measured physiological parameters being indicative for the fertility status of the mammal to predict the fertility status, and optionally taking into account additionally measured physiological or fitness parameters to adjust the fertility status prediction,
- judging 1210 by the base station 200 physiological parameters being received with the received data to predict a fertility status, and optionally taking into account additionally measured physiological or fitness parameters and/or environmental data gathered from a mobile device 300 and/or a wearable 350 to adjust the fertility status prediction, and/or
- displaying 1300 measured parameters and/or a fertility status via the base station 200, at least one mobile device 300 and/or a wearable 350.

### List of reference signs

1 intra-vaginal measuring system
10 cervix
20 data
23 menstrual phase
24 follicular phase
25 ovulation
26 luteal phase
100 intra-vaginal measuring device
110 sensor unit
112 temperature sensor
114 cervix mucus-sensor
114e electrode
116 optical sensor
120 active magnetic communication unit (transmitter or transceiver)
122 magnetic inductive antenna
124 transmission range
130 power storage unit (battery)
140 data storage unit
150 MCU
200 base station
210 display
220 active magnetic communication unit (receiver or transceiver)
230 wireless communication interface (e.g. BLE)
240 inductive charging unit
250 MCU
300 mobile device
310 display
350 wearable
400 cloud based storage
1000 method
1100 determining intra-vaginal insertion
1110 measuring physiological parameter(s)
1115 judging the fertility status
1120 transmit data
1200 receive data
1210 judging the fertility status
1300 displaying
di inner diameter
do outer diameter
T temperature
T1 first temperature (about 36.5°C)
T2 second temperature (about 37°C).

## Claims

1. An intra-vaginal measuring device (100) for determining a fertility status,
the measuring device (100) being substantially ring shaped and configured to be placed around a mammal's cervix, wherein the measuring device (100) includes
a sensor unit (110), wherein the sensor unit (110) is configured to measure at least one physiological parameter being indicative for the fertility status of the mammal; and
an active magnetic communication unit (120), wherein the active magnetic communication unit (120) is configured to transmit data (20) being based on the at least one measured physiological parameter from inside the vagina to a receiving base station (200) outside the mammal's body.

2. The intra-vaginal measuring device (100) according to claim 1, wherein the sensor unit (110) is configured to measure at least two physiological parameters being indicative for the fertility status, wherein the sensor unit (110) is optionally configured to measure intra-vaginally at least one of the following physiological parameters:
a body temperature,
a pH-value of the vaginal environment;
a dielectric constant of the cervix mucus;
a frequency dependent impedance of the cervix mucus;
a polarity of the cervix mucus;
a magnetic permeability of the cervix mucus;
at least one optical property of the cervix mucus, such as transmittance, reflectance, absorption, color and/or refraction;
a viscosity of the cervix mucus;
a heat conductivity of the cervix mucus;
a heat capacity of the cervix mucus, and/or an oxygen content of the mucous membrane.

3. The intra-vaginal measuring device (100) according to any one of claims 1 or 2, wherein the sensor unit (110) is further configured to measure additional physiological or fitness parameters, including at least one of
a heart rate;
a blood oxygen saturation;
a motional activity;
a heart rate variability;
a respiratory rate, and/or
a blood pressure.

4. The intra-vaginal measuring device (100) according to any one of claims 1 to 3, further including a microcontroller unit, MCU, (150),
the MCU (150) being configured to judge the measured physiological parameters being indicative for the fertility status of the mammal to predict a fertility status, wherein the
MCU (150) may take into account additionally measured physiological or fitness parameters to adjust the fertility status prediction.

5. The intra-vaginal measuring device (100) according to any one of claims 1 to 4, further including
a data storage unit (140), the data storage unit (140) being configured to store measured data and/or a predicted fertility status, and/or
a power storage unit (130), which may be rechargeable, wherein the intra-vaginal measuring device (100) may further include an inductive charging unit for wirelessly recharging the power storage unit (130).

6. The intra-vaginal measuring device (100) according to any one of claims 1 to 5, wherein the intra-vaginal measuring device (100) is configured to increase a measuring rate of the sensor unit (110) with advancing first menstrual cycle half and/or reducing the measuring rate after ovulation was detected.

7. The intra-vaginal measuring device (100) according to any one of claims 1 to 6, wherein
the active magnetic communication unit (120) operates at frequencies in the range of 9 MHz to 14 MHz, or in a range of about 13 MHz, and/or wherein
the active magnetic communication unit (120) has a maximum transmission range (124) being in a range from 50 cm to 120 cm, or in a range from 70 cm to 110 cm, or in a range from 80 cm to 100 cm.

8. The intra-vaginal measuring device (100) according to any one of claims 1 to 7, further including a magnetic inductive antenna (122), which magnetic inductive antenna (122) is arranged circumferentially within the ring shape of the intra-vaginal measuring device (100).

9. A base station (200) for determining a fertility status, the base station (200) including
an active magnetic communication unit (220), which is configured to receive data (20) transmitted from the active magnetic communication unit (120) of the intra-vaginal measuring device (100) according to any one of claims 1 to 8, and
a microcontroller unit (250), wherein the microcontroller unit (250) is configured to judge physiological parameters being received with the received data (20) to predict a fertility status, wherein the microcontroller unit (250) may take into account additionally measured physiological or fitness parameters and/or environmental data gathered from a mobile device (300) and/or a wearable (350) to adjust the fertility status prediction.

10. A intra-vaginal measuring system (1) for determining a fertility status, the system (1) including
at least one intra-vaginal measuring device (100) according to any one of claims 1 to 8, and
a base station (200), wherein the base station includes an active magnetic communication unit (220), which is configured to receive data (20) transmitted from the active magnetic communication unit (120) of the intra-vaginal measuring device (100).

11. The intra-vaginal measuring system (1) according to claim 10, wherein the base station (200) includes a microcontroller unit (250), wherein
the microcontroller unit (250) being configured to judge physiological parameters being received with the received data (20) to predict a fertility status, and wherein the
microcontroller unit (250) may take into account additionally measured physiological or fitness parameters and/or environmental data gathered from a mobile device (300) and/or a wearable (350) to adjust the fertility status prediction.

12. The intra-vaginal measuring system (1) according to any one of claims 10 or 11, wherein
the base station (200) is configured to display measured parameters and/or a fertility status, and/or wherein
the base station (200) is configured to forward the received data (20) and/or a fertility status to at least one mobile device (300) and/or to a cloud based storage (400).

13. The intra-vaginal measuring system (1) according to any one of claims 10 to 12, wherein
the base station (200) includes an inductive charging unit (240) for wirelessly recharging the power storage unit (130) of at least one intra-vaginal measuring device (100).

14. A method (1000) for determining a fertility status of a mammal using an intra-vaginal measuring device (100) according to any one of claims 1 to 12, the method including
determining (1100), using the sensor unit (110) that the intra-vaginal measuring device (100) is intra-vaginally inserted;
measuring (1110), using the sensor unit (110) at least one physiological parameter being indicative for the fertility status of the mammal and optionally additionally at least one physiological or fitness parameter, and
transmitting (1120), using the active magnetic communication unit (120) data (20) being based on the at least one measured physiological parameter from inside the vagina to a receiving base station (200) outside the mammal's body.

15. The method (100) according to claim 14, further comprising
judging (1115), by the intra-vaginal measuring device (100), the measured physiological parameters being indicative for the fertility status of the mammal to predict the fertility status, and optionally taking into account additionally measured physiological or fitness parameters to adjust the fertility status prediction, and/or
judging (1210), by the base station (200) physiological parameters being received with the received data (20) to predict a fertility status, and optionally taking into account additionally measured physiological or fitness parameters and/or environmental data gathered from a mobile device (300) and/or a wearable (350) to adjust the fertility status prediction, wherein the method (1000) may further include
displaying (1300) measured parameters and/or a fertility status via the base station, at least one mobile device, and/or a wearable.
